Europäisches Patentamt

European Patent Office

Office européen des brevets

(11) Numéro de publication: **0 271 380 B1**

(12) **FASCICULE DE BREVET EUROPEEN**

(45) Date de publication de fascicule du brevet: **22.09.93** (51) Int. Cl.⁵: **C12M 1/36**, C12P 7/06

(21) Numéro de dépôt: **87402543.0**

(22) Date de dépôt: **10.11.87**

(54) **Procédé de prévision et de contrôle en ligne des fermentations alcooliques.**

(30) Priorité: **12.11.86 FR 8615719**

(43) Date de publication de la demande:
**15.06.88 Bulletin 88/24**

(45) Mention de la délivrance du brevet:
**22.09.93 Bulletin 93/38**

(84) Etats contractants désignés:
**CH DE ES IT LI**

(56) Documents cités:
EP-A- 0 089 225
DE-A- 1 935 818
DE-B- 2 744 769
GB-A- 1 434 613

**BIOTECHNOLOGY AND BIOENGINEERING,
vol. 23, no. 2, 1981, pages 405-417, John
Wiley & Sons, Inc., New York, US; L. NYESTE
et al.: "Automated fermentation equipment.
II. Computer-fermentor system"**

(73) Titulaire: **INSTITUT NATIONAL DE LA RECHER-
CHE AGRONOMIOUE (INRA)**
**147, rue de l'Université**
**F-75341 Paris Cédéx 07(FR)**

Titulaire: **CENTRE NATIONAL DU MACHINI-**

**SME AGRICOLE, DU GENIE RURAL, DES
EAUX ET DES FORETS (CEMAGREF)
Domaine de Lavalette Avenue du Val de
Montferrand
F-34033 Montpellier Cédex(FR)**

(72) Inventeur: **Barre, Pierre**
**90 rue des Lavandes**
**F-34980 St Gely du Fesc(FR)**
Inventeur: **Chabas, Jacques**
**Lotissement de l'Aqueduc No. 2, Rue Croix
de Lavit**
**F-34100 Montpellier(FR)**
Inventeur: **Corrieu, Georges**
**2 Avenue des Combattants**
**F-78220 Viroflay(FR)**
Inventeur: **Davenel, Armel**
**17 rue Paul Gauguin**
**F-34170 Castelnau-le-Lez(FR)**
Inventeur: **El Haloui, Noureddine**
**369 rue Jules Guesde**
**F-59650 Villeneuve D'Ascq(FR)**
Inventeur: **Grenier, Pierre**
**16 rue Toiras**
**F-34000 Montpellier(FR)**

Inventeur: **Navarro, Jean-Marie**
**2429 Avenue du Père Soulas Villa 133**
**F-34100 Montpellier(FR)**
Inventeur: **Picque, Daniel**
**43 Chaussée de l'Hotel de Ville**
**F-59650 Villeneuve d'Ascq(FR)**
Inventeur: **Sablayrolles, Jean-Marie**
**15 rue des Volontaires**
**F-34000 Montpellier(FR)**
Inventeur: **Sevila, François**
**460 rue du Rioch de Boutonnet**
**F-34100 Montpellier(FR)**
Inventeur: **Vannobel, Claude**
**10 Lotissement de Chateaubon**
**F-34100 Montpellier(FR)**

(74) Mandataire: **Phélip, Bruno et al**
**c/o Cabinet Harlé & Phélip 21, rue de La**
**Rochefoucauld**
**F-75009 Paris (FR)**

## Description

La présente invention est du domaine des fermentations agricoles. Elle vise plus précisément un procédé de prévision et de contrôle in situ, c'est-à-dire en ligne, des fermentations alcooliques.

On connaît de nombreux procédés agro-alimentaires et agro-industriels dans lesquels sont impliquées des fermentations alcooliques, en pratique pour la production des boissons alcoolisées ainsi que de l'éthanol, du bioéthanol, à partir de substrats à base de sucre ou de céréales. Ces procédés permettent de réaliser des fermentations alcooliques dans des conditions plus ou moins performantes, mais jamais optimales. Ceci est dû, en particulier, au fait qu'il n'existe pas de moyen d'évaluation en ligne soit de l'avancement du procédé, lorsqu'il s'agit de production discontinue, par exemple, la bière, le vin, le cidre, le bioéthanol et analogues, soit de la stabilité et du point d'équilibre du procédé lorsqu'il s'agit de productions en continu, par exemple du bioéthanol.

En l'absence de tels moyens, on procède dans les milieux professionnels concernés de la brasserie, de l'oenologie et analogues, à des mesures des différents paramètres de la fermentation, tels que la densité, l'indice de réfraction et analogues, sur des échantillons, ces mesures étant complétées ultérieurement par des analyses chimiques d'autres paramètres, tels que les teneurs en sucre, en éthanol, en composés volatils, l'indice d'acide et analogues. En brasserie, par exemple, alors que l'ensemble du procédé de production de bière est automatisé, en particulier le brassage, notamment au niveau de la production du moût qui va être fermenté, la conduite de la fermentation se fait de façon empirique, par paliers successifs à température contrôlée pendant des durées généralement préétablies, faute des moyens évoqués ci-dessus. En oenologie, les mesures empiriques sont encore plus importantes. On notera, par exemple que le contrôle de la température est encore peu répandu, tandis que la réfrigération des cuves, par exemple par ruissellement d'eau, mise en oeuvre d'un groupe réfrigérant mobile et analogues, se fait au coup par coup en fonction de l'élévation éventuelle de la température des moûts en cours de fermentation. Il existe donc un réel besoin d'un procédé permettant de normaliser ces opérations tout en fournissant une information précise sur l'avancement ou la stabilité de la fermentation alcoolique.

A titre de référence illustrant la technique antérieure, on peut citer le brevet GB-A-1 434 613 qui décrit le contrôle de procédés biologiques à partir de mesures en ligne sélectionnées, permettant la détermination de variables non mesurables, qui traduisent l'état physiologique et biologique des procédés. Les actions s'effectuent sur certaines variables mesurées afin d'agir sur les grandeurs calculées et d'aboutir à l'optimisation des procédés.

L'objet effectif de ce brevet antérieur est le contrôle de procédés biochimiques catalysés par des enzymes, liées ou non à des cellules, et en particulier le contrôle de la multiplication cellulaire et du métabolisme dans des cultures immergées. Les moyens et les paramètres proposés ne conviennent pas au contrôle de la fermentation alcoolique, car ils sont essentiellement destinés à des réactions aérobies, alors que les fermentations alcooliques mises en jeu dans la présente invention sont anaérobies.

Le brevet EP-A-0 089 225 concerne le domaine général de la fermentation alcoolique et a pour objet un procédé permettant, par mesure du pH de la culture, d'agir sur l'injection d'oxygène dans le milieu et sur la température afin d'obtenir une croissance optimale de la biomasse (levure) apte à la fermentation alcoolique. Ainsi, il propose un procédé de contrôle applicable au développement initial de la biomasse, mais il n'enseigne aucun moyen pour contrôler la phase proprement dite de fermentation alcoolique, laquelle implique la production d'éthanol et de $CO_2$ par dégradation des sucres.

Le brevet DE-B-2 744 769 a pour objet un procédé pour commander un procédé de fermentation assurant la production de boissons fermentées. Il se base sur la mesure du volume de $CO_2$ dégagé et se propose d'agir, selon des règles de conduite pré-établies, sur des variables d'action, telles que la température et la pression. Ce brevet antérieur contient un enseignement très général basé sur la mise à profit de la loi de Gay-Lussac, qui est bien connue dans les processus de fermentation, mais il ne décrit pas les moyens techniques permettant de prédéterminer la concentration en sucres et la concentration en éthanol, qui sont des paramètres essentiels pour traduire l'évolution de la fermentation alcoolique.

Le brevet DE-A-1 935 818 décrit un procédé et une installation pour le contrôle d'un processus de fermentation, en particulier lors de production de bière. Le contrôle est basé sur la mesure du poids spécifique du milieu de fermentation. L'automatisme, par action sur la température de fermentation, concerne le suivi d'une courbe pré-établie d'évolution du poids spécifique. Comme dans le cas du brevet précédent (DE-B-2 744 769), ce brevet DE-A-1 935 818 n'enseigne pas les moyens de prédéterminer la concentration en sucres et la concentration en éthanol.

Les références bibliographiques illustrant l'état de la technique montrent que l'on s'efforce encore de trouver des moyens permettant de contrôler efficacement les processus de fermentation alcoolique, en mettant à profit les possibilités offertes par l'informatique.

L'invention a pour objet un tel procédé de contrôle, qui utilise des paramètres physiques permettant d'aboutir à la détermination des concentrations en éthanol et en sucres résiduels.

Un objet de la présente invention est donc de fournir un procédé permettant à la fois de prévoir l'état final, d'évaluer l'avancement de la fermentation, d'établir les cinétiques de fermentation et de formuler un diagnostic sur les conditions de déroulement du processus de fermentation alcoolique.

Un autre objet de la présente invention est de fournir un procédé permettant, à partir de la détermination de paramètres distinctifs de la fermentation alcoolique, de contrôler ladite fermentation, afin que celle-ci puisse se dérouler dans des conditions optimales.

La présente invention a donc pour objet un procédé de prévision et de contrôle in situ des fermentations alcooliques, caractérisé en ce que l'on mesure à intervalles de temps donnés au moins un paramètre choisi parmi la densité du milieu de fermentation, l'indice de réfraction dudit milieu, le volume de gaz carbonique produit par ledit milieu et la perte de poids du milieu de fermentation, en ce que l'on détermine, à partir de ces paramètres, les concentrations en éthanol et en sucres résiduels, concentrations à partir desquelles on peut déterminer l'avance et l'évolution de la fermentation alcoolique, diagnostiquer les évolutions incorrectes (arrêts ou accidents de fermentation) et contrôler cette dernière de façon à corriger toute évolution incorrecte du déroulement de ladite fermentation.

Dans un mode de réalisation, on détermine la cinétique, instantanée ou globale, de la fermentation, par exemple la vitesse de consommation des sucres, la vitesse de production d'éthanol et la vitesse de production de $CO_2$.

A titre d'exemple, la détermination de la cinétique instantanée ou globale de la fermentation est mise à profit pour contrôler automatiquement l'évolution de la fermentation, en particulier en provoquant des modifications appropriées de la température.

Ainsi, selon un mode de réalisation préféré du procédé selon la présente invention, le contrôle de l'évolution de la fermentation s'effectue, en fonction des déterminations obtenues des paramètres choisis, par modification appropriée de la température du milieu de fermentation.

Ainsi qu'il a déjà été indiqué précédemment, et comme l'illustrera avec plus de détails la description qui suit, la présente invention comporte des caractéristiques originales et avantageuses, parmi lesquelles:

(1) le procédé de contrôle est basé sur les mesures possibles de quatre paramètres physiques différents, dont les intercorrélations ont été déterminées.

(2) ces mesures permettent de déterminer les deux grandeurs biologiques essentielles pour traduire l'évolution du procédé de fermentation alcoolique, à savoir la concentration en sucres et la concentration en éthanol. Les corrélations établies pour assurer ces prédéterminations sont de forme linéaire (donc très simples).Les moyens informatiques proposés permettent un tracé et à tout instant .

(3) le procédé comporte également la détermination et le tracé en temps réel de la cinétique fermentaire.

(4) parallèlement, il comporte une analyse de l'état d'avancement de la fermentation avec diagnostic des accidents éventuels (arrêts, accidents, etc...).

(5) le procédé de contrôle permet d'agir, in fine, sur la température de fermentation, de manière que la loi de pilotage soit choisie pour chaque type de produit (type de vin, type de bière) en fonction de l'évolution de la cinétique de fermentation.

De telles caractéristiques sont particulièrement intéressantes dans la pratique et n'étaient enseignées par aucun des documents antérieurs mentionnés au début du présent mémoire descriptif.

D'autres avantages et caractéristiques de la présente invention apparaîtront à la lecture de la description non limitative suivante, d'un procédé pour la précision et le contrôle in situ des fermentations alcooliques, en référence aux dessins annexés dans lesquels:

Fig. 1 est une représentation schématique d'une forme de réalisation de dispositifs permettant la mise en oeuvre du procédé selon l'invention.

Fig. 2 représente la corrélation expérimentale entre la perte de poids du milieu de fermentation et le volume de gaz carbonique dégagé pour des teneurs initiales en sucre dudit milieu de fermentation, comprises entre 160 et 260 g/l.

Fig. 3 représente des courbes de corrélation entre la densité et l'indice de réfraction pour des moûts de fermentation présentant une concentration initiale en sucre comprise entre 160 et 260 g/l.

Fig. 4 représente des courbes de corrélation entre la densité et le volume de $CO_2$ produit pour des moûts de fermentation présentant une concentration initiale en sucre comprise entre 160 et 260 g/l.

Fig. 5 montre la corrélation entre la concentration en éthanol dans le milieu de fermentation et le volume de gaz carbonique dégagé pour des teneurs initiales en sucre comprise entre 160 et 260 g/l.

Fig. 6 montre la corrélation entre la concentration en sucre consommé dans le milieu de fermentation et le volume de $CO_2$ dégagé pour des teneurs initiales en sucre comprise entre 160 et 260 g/l.

Fig. 7 représente des courbes de corrélation entre la densité et la concentration en alcool dans des moûts pour des concentrations initiales en sucre comprises entre 160 et 260 g/l.

Fig. 8 représente une courbe illustrant la corrélation entre la densité et la concentration en sucre résiduel dans les moûts pour des concentrations initiales en sucre comprises entre 160 et 260 g/l.

Fig. 9 représente une comparaison des résultats expérimentaux (notés +) et des grandeurs prédites-(courbe en trait plein) en utilisant la corrélation basée sur la mesure du volume de $CO_2$ dégagé.

Fig. 10 représente une comparaison des résultats expérimentaux (notés +) et des grandeurs prédites (courbe en trait plein) de la concentration en sucres consommés en fonction de la durée de fermentation,en utilisant la corrélation basée sur la mesure de volume de $CO_2$ dégagé et la concentration initiale en sucres.

Fig. 11 illustre la cinétique de la production d'éthanol selon une courbe d'évolution de la vitesse de production d'éthanol en fonction du temps, en se basant sur la corrélation de la figure 3.

Fig. 12 est une illustration de la cinétique de dégagement de $CO_2$ selon une courbe d'évolution de la vitesse de production de $CO_2$,en fonction du temps obtenu grâce à la mesure du volume de $CO_2$.

Fig. 13 est une vue schématique d'une cuve d'expérimentation en cave du procédé selon la présente invention.

Figs. 14A et 14B représentent respectivement en vue avant et arrière une cuve industrielle pour la mise en oeuvre du procédé de la présente invention.

Fig. 15 représente les valeurs des teneurs en éthanol calculées en pourcentage volumique, en fonction des valeurs des teneurs en éthanol mesurées par l'analyse chimique, au cours d'une première fermentation de moûts de cépages mélangés,dans la cuve décrite aux figures 14A et 14B, remplie à 45 hectolitres,les signes différents employés sur le graphique permettant de distinguer le calcul fait à partir de la mesure d'indice réfractométrique de celui fait à partir de la mesure de masse volumique du moût en fermentation.

Fig. 16 et figure 17 sont respectivement des graphiques identiques à celui de la figure 15,établis sur d'autres fermentation de moûts de cépages mélangés.

Fig. 18 représente la courbe des concentrations en sucres calculées par mesure de dégagement gazeux et celle des concentration en sucres analysées, en fonction du temps dans un moût en fermentation.

Fig. 19 représente le graphique des concentrations en sucres calculées par mesure de dégagement gazeux, portées en fonction des concentrations mesurées par l'analyse chimique, dans un moût en fermentation.

Figs. 20 et 21 représentent des graphiques identiques à celui de la figure 19, mais sur d'autres fermentations.

Fig. 22 représente en particulier des courbes d'évolution de la densité calculée à partir de mesures de pression en haut et bas de cuve dans sa partie immergée, ainsi que la densité mesurée à l'aide d'un aréomètre à partir d'une prise d'échantillon, en fonction du temps, au cours d'une fermentation en cave.

Fig. 23 représente les courbes d'évolution du gaz carbonique dégagé et de la température, respectivement, en fonction du temps à l'intérieur d'un bio-réacteur piloté au moyen du dispositif de la figure 1.

Fig. 24 présente les mêmes courbes que sur la figure 23 mais sans aucun contrôle de la fermentation.

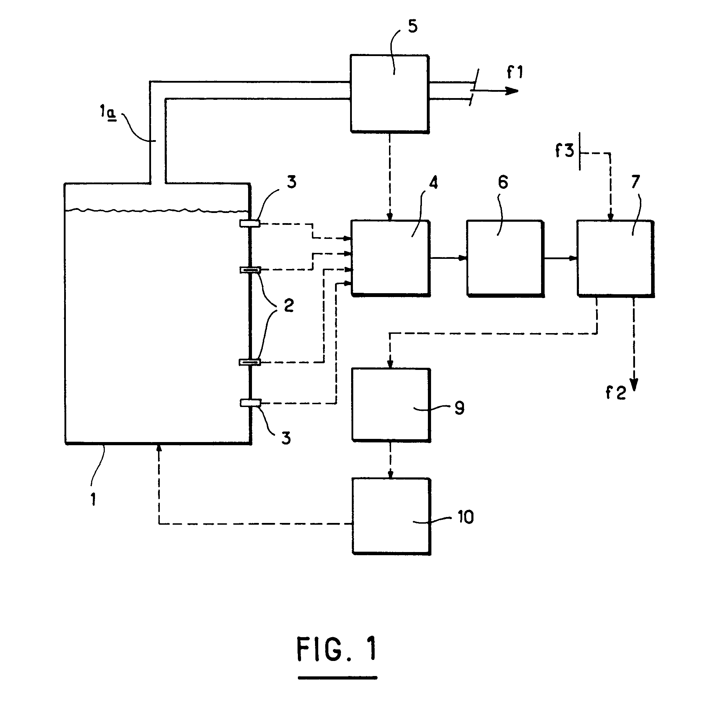

Comme représenté sur la figure 1, du bio-réacteur 1 renfermant un moût de fermentation, se dégage par la conduite 1a du gaz carbonique qui est mesuré au moyen d'un compteur volumétrique 5 avant de se dégager selon la flèche f1.

Les données mesurées par ce compteur volumétrique 5 sont transmises à l'interface électronique de mesure 4 à laquelle aboutissent également les données de température et de pression mesurées dans la cuve du bioréacteur 1 respectivement par les capteurs de température 2 et les capteurs de pression 3. L'interface électronique de mesure 4 transmet ces données à l'unité de traitement en ligne 6 constitué par exemple d'un micro-calculateur et de logiciels de mise en oeuvre, cette unité de traitement en ligne 6 étant connectée à une unité périphérique 7 associée, par exemple, à un écran clavier, à une unité de traitement de disquettes, à une imprimante et analogues. Cette unité périphérique 7 coopère, d'une part, selon f2 avec l'opérateur en lui fournissant des informations de diagnostic, ledit opérateur pouvant selon f3 introduire des données initiales dans ladite unité périphérique 7, et, d'autre part, avec une interface électronique de commande 9 destinée à contrôler des moyens actionneurs 10 régulant par exemple la température du bio-réacteur 1.

Comme exposé ci-dessus, le procédé de prévision et de contrôle des fermentations alcooliques de l'invention est basé sur la mesure de plusieurs grandeurs physiques représentatives de la fermentation alcoolique à savoir: la densité du milieu de fermentation; l'indice de réfraction du milieu de fermentation; le volume de gaz carbonique produit; la perte de poids du milieu de fermentation, du fait de la transformation du sucre en éthanol et en gaz carbonique qui se dégage.

L'analyse des résultats obtenus dans les différentes courbes des figures conduit aux conclusions suivantes:

Les quatre paramètres considérés sont d'excellents indicateurs du déroulement de la fermentation alcoolique. En tenant compte des conditions expérimentales, à savoir la concentration initiale en sucre du moût, de la température de fermentation contrôlée ou évoluant librement, de l'inoculum ainsi que d'autres paramètres, il est possible d'établir des corrélations entre un ou plusieurs de ces paramètres mesurés en ligne et les concentrations en éthanol et en sucres résiduels dans les moûts.

D'autre part il existe une corrélation très étroite entre la perte de poids et le volume du gaz carbonique dégagé, qui peut être exprimé en litre ou en g/l de moût fermenté. En fait les deux mesures réalisées simultanément n'apporteraient pas plus d' information qu'une mesure unique.

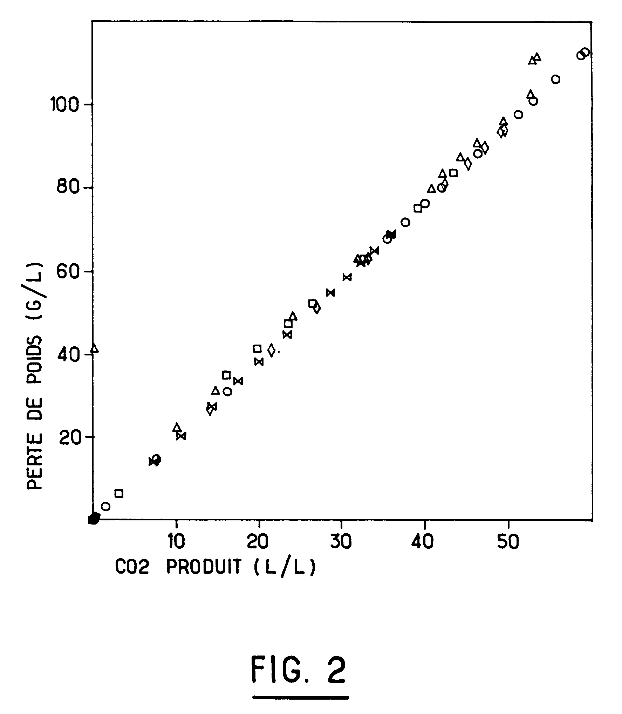

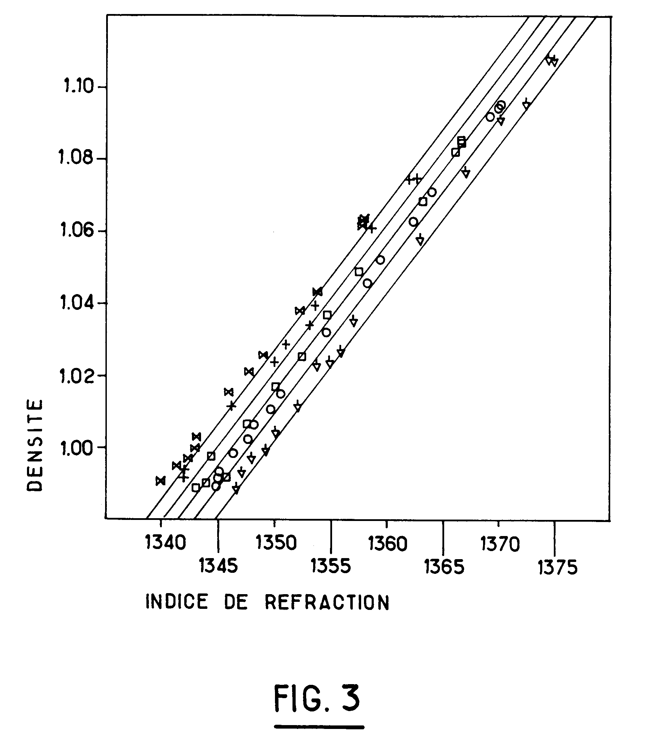

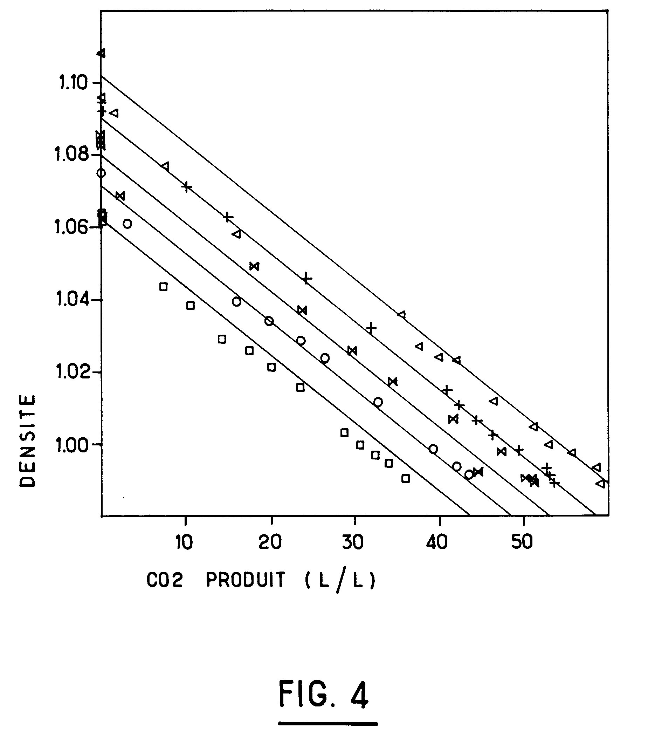

En ce qui concerne les figures 3 et 4, on remarquera, qu'en relation avec la figure 2, elles établissent les combinaisons existant entre les quatre paramètres physiques mesurés, spécifiés également ci-dessus.

Par ailleurs les figures 3 et 4 montrent l'existence de relations linéaires entre respectivement les concentrations en éthanol produit et en sucre consommé et le volume de $CO_2$ dégagé, ces relations étant de la forme $Y = a + bX$. Les résultats expérimentaux ont permis l'identification des paramètres a et b pour lesquels on dispose donc de valeurs numériques parfaitement définies. Pour la concentration en éthanol (Eth), la relation s'écrit:

$$Eth = a + b.VCO_2,$$

la précision étant de ± 2,73 g/l.

La prise en compte au sein du modèle de la température de fermentation améliore généralement la précision de la prédiction, à savoir ± 2,4 g/l.

En ce qui concerne la concentration en sucres résiduels (Sr), la relation s'écrit:

$$Sr = a + bVCO_2 + CSo,$$

So étant la concentration initiale en sucres, tandis que la précision est de ± 7,8 g/l. Si la température de fermentation est prise en compte dans le modèle la précision de la prédiction est légèrement améliorée, à savoir ± 7,7 g/l. Cette amélioration est plus sensible à savoir ± 6 g/l, si la densité est également introduite dans le modèle.

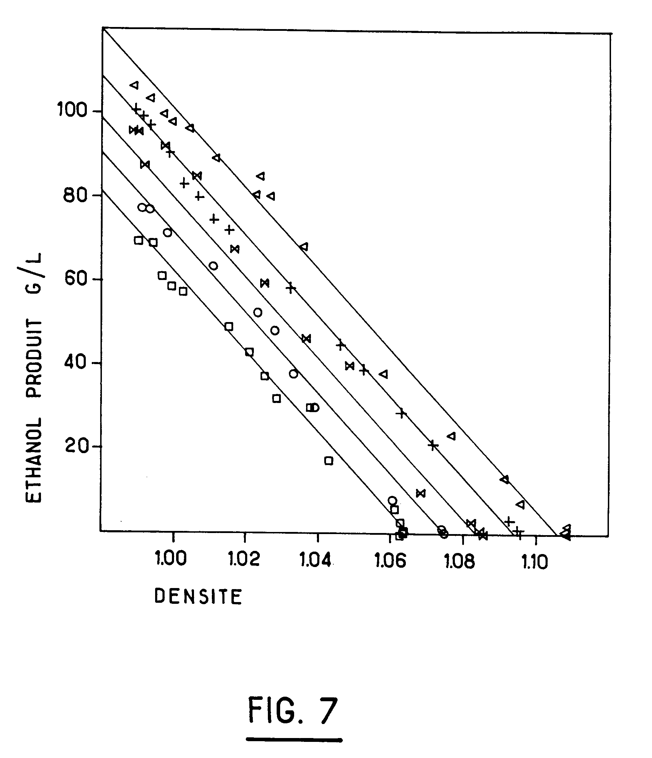

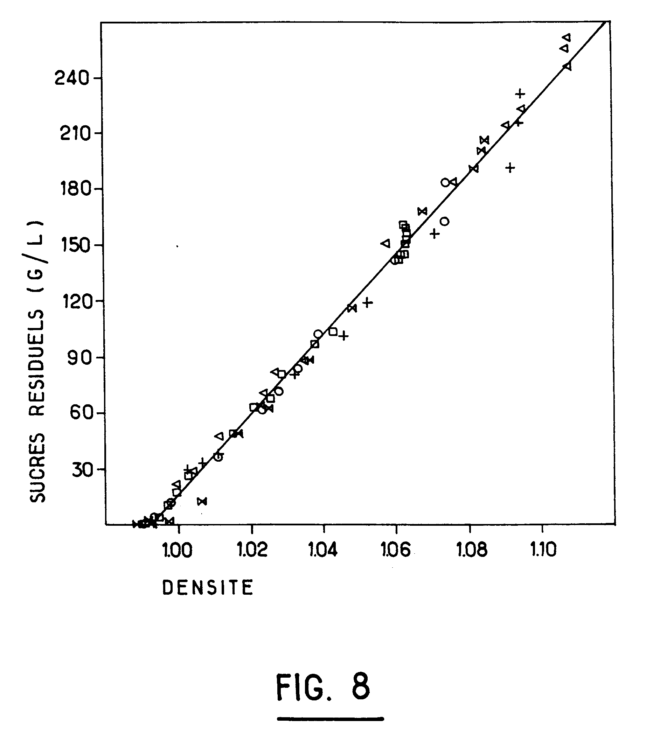

Les résultats des figures 7 et 8 , démontrent qu'outre la mesure du $CO_2$, il est possible d'utiliser la densité du milieu de fermentation pour déterminer au préalable les concentrations en éthanol et en sucres résiduels. Cette règle peut être étendue, comme le montrent les figures 2, 3, 4, à l'indice de réfraction et à la perte de poids.

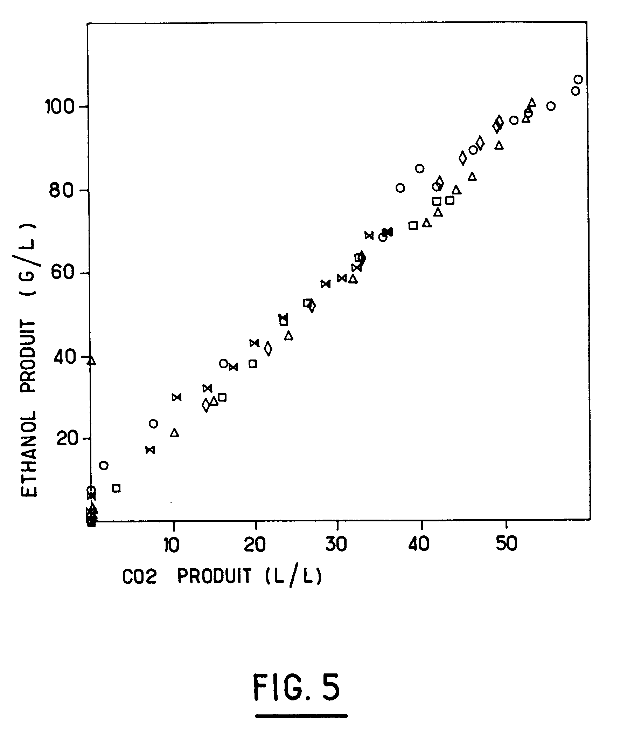

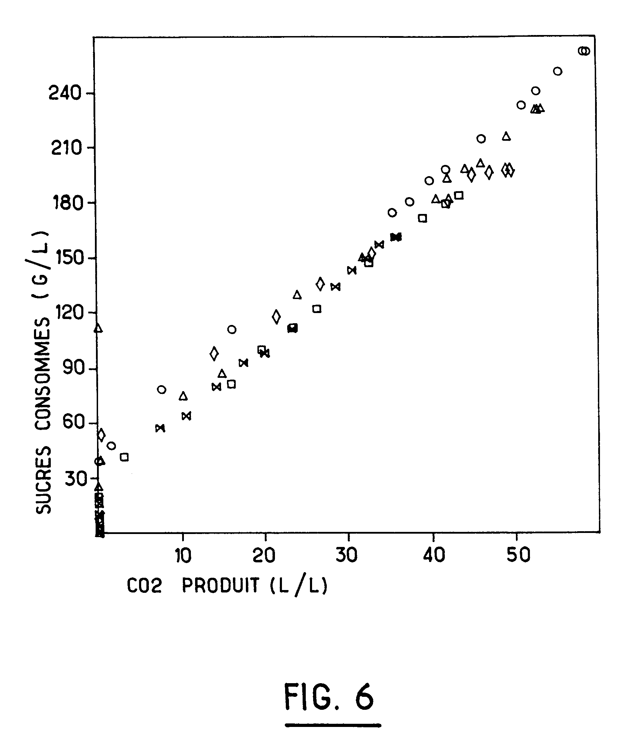

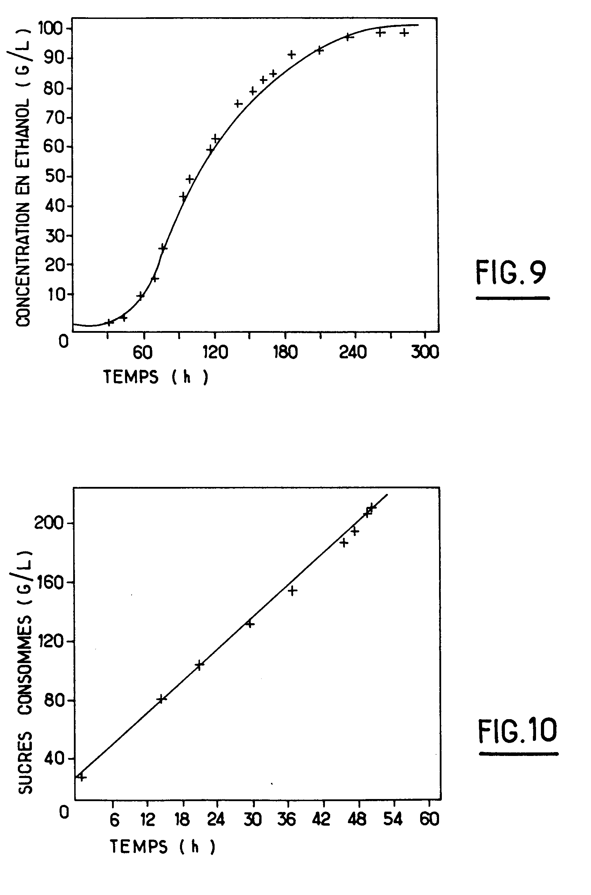

Sur la figure 9, on a établi une comparaison des résultats expérimentaux (noté +) et des grandeurs prédites (courbe en trait plein) en utilisant la corrélation basée sur la mesure du volume de $CO_2$ dégagé, en relation avec les résultats de la figure 5. Par ailleurs la figure 10 établit une comparaison des résultats expérimentaux (noté +) et des grandeurs prédites (courbe en trait plein) de la concentration en sucre consommé en fonction de la durée de fermentation, en utilisant la corrélation basée sur la mesure du volume de $CO_2$ dégagé et la concentration initiale en sucre, en rapport avec la figure 6. Ces deux dernières figures démontrent clairement l'intérêt des modèles établis qui permettent de déterminer à tout instant de la fermentation alcoolique, les teneurs en sucre et en éthanol du milieu de fermentation.

De ce qui précède, on voit que les mesures réalisées en ligne et concernant les paramètres précités, mesures qui lorsqu'elles sont utilisées au sein de modèles mathématiques, permettent de prédire, avec une précision de quelques g/l (soit généralement mieux que 5%) les concentrations en éthanol et en sucres résiduels du moût au cours de la fermentation. Par rapport à la prévision de l'état final, il est possible d'exprimer en pourcentage le degré d'avancement de la fermentation. Comme trois des paramètres précités sont disponibles, à savoir la quantité totale de $CO_2$ produit, l'éthanol formé et le sucre consommé, un recoupement est possible afin d'améliorer l'expression du résultat.

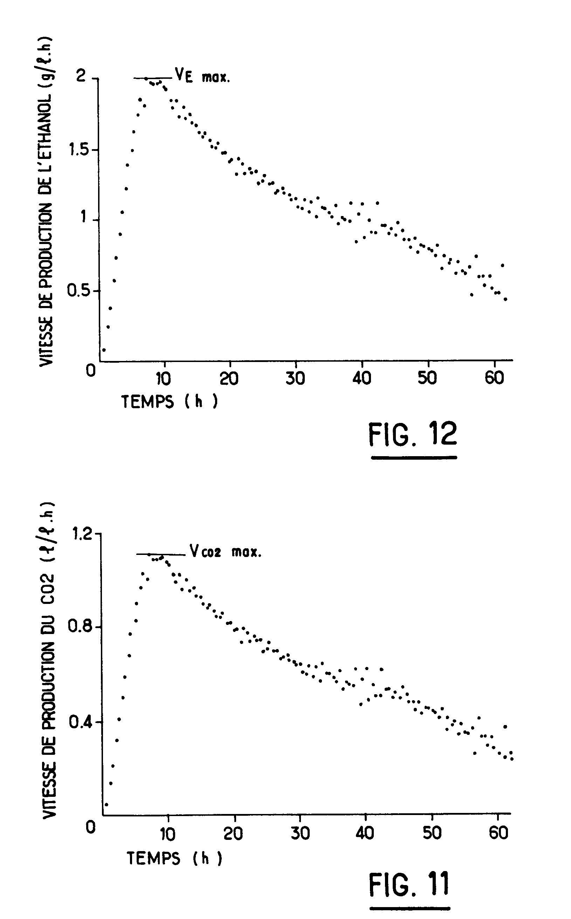

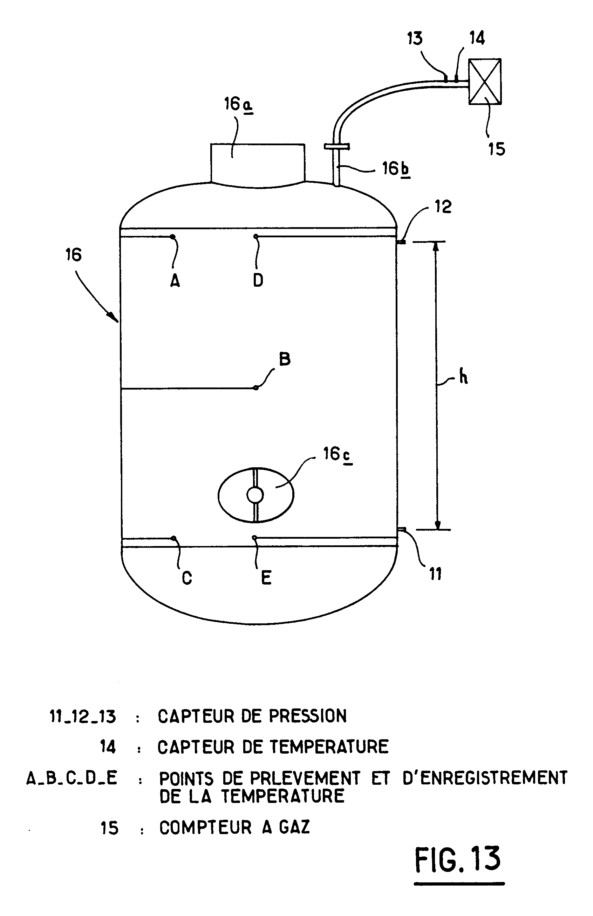

Grâce aux mesures effectuées ($CO_2$) et aux prévisions (concentration en éthanol et en sucres résiduels), il est possible en se référant au temps de déterminer les vitesses de production de $CO_2$ et d'éthanol et la vitesse de consommation des sucres. Les cinétiques représentées aux figures 12 et 11 constituent un outil idéal pour gérer les fonctions alarmes et diagnostics, par exemple en mettant en oeuvre le dispositif de contrôle de la figure 1.

En outre, par référence aux expériences accumulées, les cinétiques, établies à partir des mesures et des déterminations prévisionnelles desdits paramètres, permettent d'indiquer à l'opérateur si le déroulement de la fermentation est normal ou hors norme. Par exemple, les diagnostics suivants sont envisageables:

6

fermentation normale, fermentation trop lente, fermentation arrêtée prématurément, c'est-à-dire avant épuisement des sucres, fermentation trop rapide et autres anomalies de fermentation. Grâce aux données que lui communique le dispositif de la figure 1, l'opérateur peut procéder à une action de refroidissement au moment le plus adéquat pour la mettre en oeuvre.

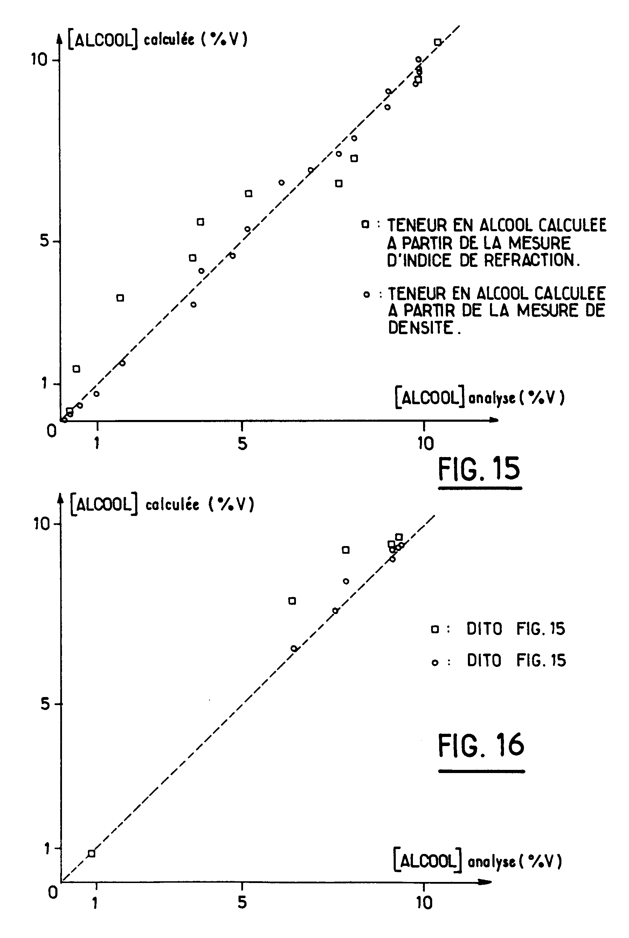

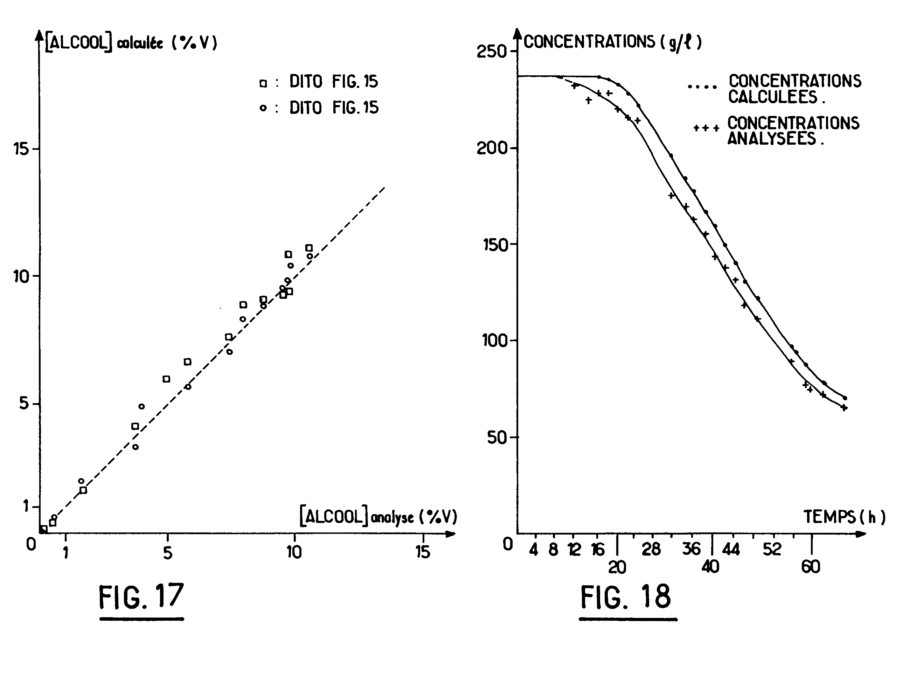

D'autres expérimentations, dont les résultats sont indiqués sur les figures 15 à 22, ont été réalisées à l'échelle industrielle. Ces résultats ont permis de faire la preuve de la faisabilité industrielle du procédé à l'échelle de plusieurs dizaines d'hectolitres de vin. On a observé, d'une part, que l'homogénéité de la fermentation au sein de la cuve étant assez bonne il suffit d'une information globale sur l'activité fermentaire de la cuve pour l'extrapoler à chaque zone de la cuve; d'autre part, la validité de corrélations reliant la densité et/ou l'indice réfractométrique à la teneur en sucres et éthanol des moûts en fermentation montre que les expérimentations dont les résultats sont indiqués aux figures 2 à 12 sont extrapolables à la situation industrielle à laquelle doit s'appliquer le procédé de la présente invention.

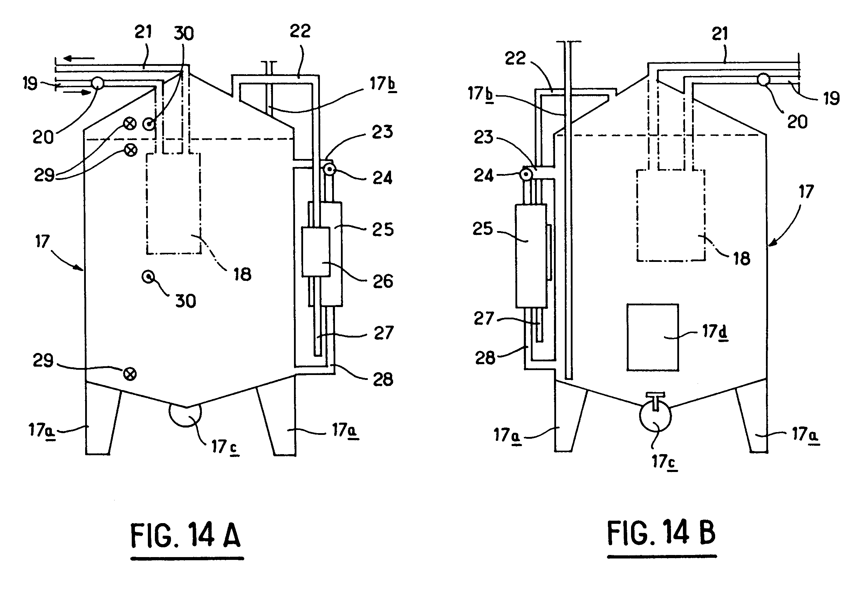

Pour ce faire, on a utilisé, d'une part, une cuve d'expérimentation en cave telle que représentée à la figure 13, cette cuve ayant une contenance de 30 hectolitres et, d'autre part, une cuve de 50 hectolitres plus particulièrement adaptée à être associée au dispositif de contrôle de fermentation de la figure 1, cette cuve étant représentée sur les figures 14A et 14B.

La cuve d'expérimentation en cave représentée sur la figure 13 comporte un corps 16 sur lequel sera rapporté un couvercle 16a, un piquage 16b et une vanne-vidange 16c. Le $CO_2$ dégagé par la fermentation sort du piquage en 16b pour être amené à un compteur volumétrique 15 en aval duquel sont prévus un capteur de pression 13 et un capteur de température 14. Sur le corps de cuve 16, sont également montés selon une dénivellation h des capteurs de pression 11 et 12. Les points A, B, C, D, E sont les points d'enregistrement de la température à l'intérieur du corps 16.

Dans la forme de réalisation de cuve montrée aux figures 14A et 14B, le corps 17 est monté sur une embase 17a et présente à son extrémité inférieure une vanne de vidange 17c. A l'intérieur de la cuve est monté un échangeur thermique à plaque 18 recevant par une conduite 19 relié à un groupe de réfrigération, un liquide caloporteur dont la circulation est commandée par une vanne 20. Le liquide caloporteur retourne au groupe de réfrigération par la conduite 21. A l'extrémité supérieure de la cuve 17 est prévu un piquage 17b pour un dispositif de lecture de la hauteur du moût. Le $CO_2$ produit dans la cuve est capté par une conduite 22 qui le transfère dans un compteur volumétrique 26, après quoi il est dégagé par la conduite 27. En parallèle à la cuve est monté un densimètre électronique relié à la cuve par un conduit 23 sur lequel sont montés une vanne 24 et un conduit 28. A l'intérieur de la cuve sont matérialisés en 29 des capteurs de pression et en 30 des capteurs de température. Le corps de cuve est également pourvu d'une porte 17d.

Les mesures manuelles faites sur prises d'échantillons au cours des manipulations en cave ont porté particulièrement la détermination de l'indice de réfraction et de la densité, ainsi que de la température dont l'usage est limité à la correction des paramètres physiques. Le tableau 1 suivant présente le profil des teneurs en alcool et glycérol ainsi que celui des indices de réfraction dans une cuve contenant 27 hectolitres de moût en fermentation, telle que schématisée sur la figure 13. Il s'agit dans ce cas d'un moût issu de cépages rouges, vinifié en phase liquide après "saignée".

Le tableau 2 correspond aux résultats obtenus avec une cuve renfermant 45 hectolitres de moût en fermentation, issu de cépages rouges avec chauffage de vendange, telle que représentée sur les figures 14A et 14B.

Les résultats des analyses chimiques du tableau 1 sont affectés par une conservation des échantillons de qualité hétérogène (congélation seule) et les mesures d'indice de réfraction sont assez peu précises. Par contre les résultats du tableau 2 sont très fiables, le protocole expérimental étant meilleur. On peut donc conclure qu'il existe une certaine hétérogénéité de l'activité fermentaire dans les cuves, significative devant la précision des mesures chimiques. Mais les variations de concentrations en sucre calculées sur un intervalle de temps de 10 ou 14 heures, révèlent des écarts relatifs maximum de 10% dès que la fermentation devient active dans l'ensemble de la cuve. Cette hétérogénéité est suffisamment faible pour qu'une mesure de synthèse telle que celle du dégagement gazeux ou de la densité moyenne calculée par différence de mesures de pression, reflète finalement l'activité du fermenteur.

En ce qui concerne les figures 15,16,17, il s'agit d'une extrapolation à un volume de 4500 litres de moût des corrélations établies sur micro-vinification.

Comme résultat des recherches effectuées par l'Ecole d'Oenologie de la Faculté de Pharmacie de Montpellier en collaboration avec le CEMAGREF, une corrélation a été établie reliant l'indice réfractométrique n et la masse volumique $\rho$ à la composition du moût en extrait sec non sucré (E, en g/l), sucres totaux (S en g/l), éthanol (A, en % V), glycérol (G, en g/l), et en tenant compte de la température (en °C) de la mesure.

Cette corrélation correspond aux formules suivantes:

$n = 1,3330 - 0,000103 \, (t-20) + [1,4199 - 0,00181 \, (t-20)] \times 10^{-4} \, (E+S) + [5,465 - 0,00189 \, (t-20)] \times 10^{-4} \cdot A + 1,1560 \times 10^{-4} \cdot G$ $\rho = 998,2 - 0,3 \, (t-20) + 0,3820 \, (E+S) - 1,3050 \cdot A + 0,2311 \cdot G$

Compte-tenu des hypothèses oenologiques reliant G, A, S et fixant la valeur de E, on s'est réduit à une seule inconnue qui sera A ou S; on peut ainsi admettre que 1% en volume d'éthanol formé est équivalent à la dégradation de 17,5g/l de sucres. En tenant compte de ces hypothèses, et en utilisant l'équation portant sur $\rho$ uniquement, les teneurs en éthanol ont été calculées dans le cas de fermentations ayant lieu en cave vinicole en volume de 4500 litres. Les résultats sont illustrés par les figures 15, 16 et 17. Parallèlement les résultats utilisant l'indice réfractométrique seul sont représentés. Bien que les mesures faites sur le terrain en cave vinicole soient entâchées d'une certaine erreur, surtout dans le cas de l'indice réfractométrique, la faisabilité du suivi des fermentations par mesures d'indices réfractométriques ou densité en situation industrielle est clairement démontrée.

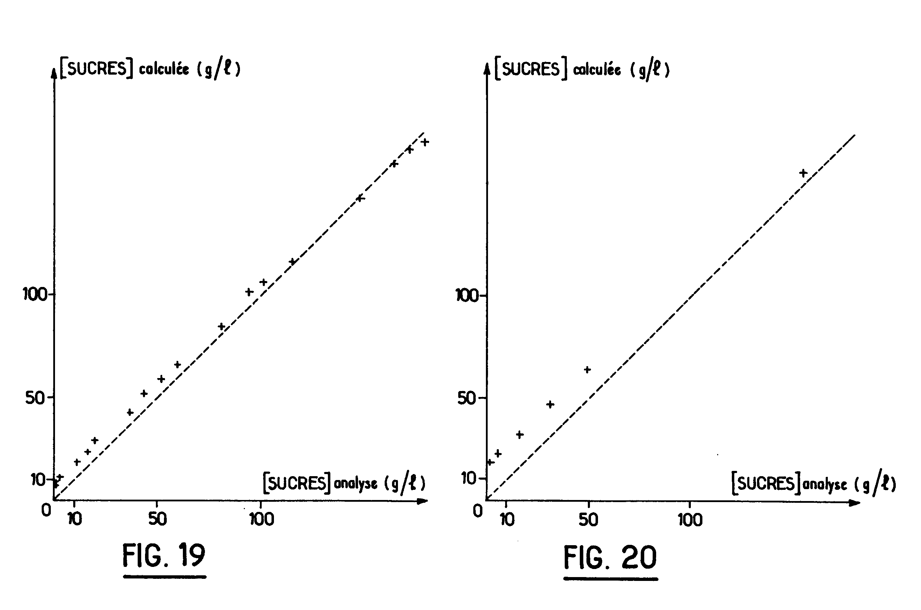

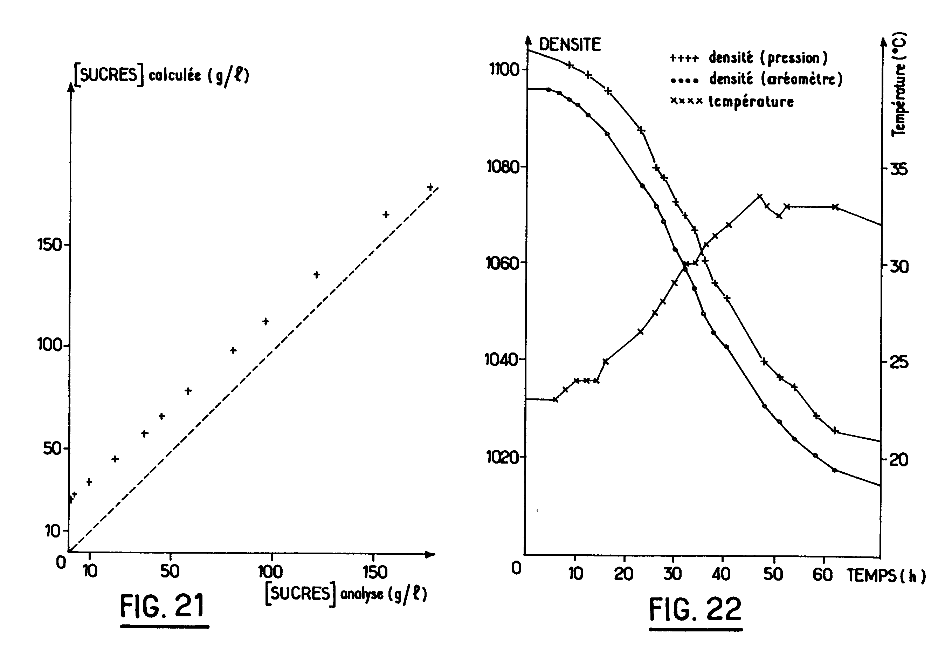

Les figures 18 à 21 montrent qu'un compteur volumétrique à gaz, par exemple du type représenté en 15 ou 25 sur les figures 13 et 14A, 14B suffit pour le calcul de la masse de gaz carbonique dégagé. Les courbes présentées sur les figures 19 à 21 montrent la linéarité de la corrélation reliant l'indication fournie par le compteur et la teneur en sucres du moût en fermentation. Les décalages observés sur les figures 20 et 21 ne mettent en cause que le fonctionnement proprement dit du compteur volumétrique mais pas le principe de la mesure; les phénomènes de condensation qui sont la cause de cette déviation de la mesure, obligent à prévoir un système complémentaire de condensation des vapeurs en amont du passage des gaz dans le compteur; au cas où ce système complémentaire ne résoudrait pas le problème, d'autres types d'instrument pourraient être utilisés avec un succès certain (débit-mètres à orifice dépressiogène et analogues).

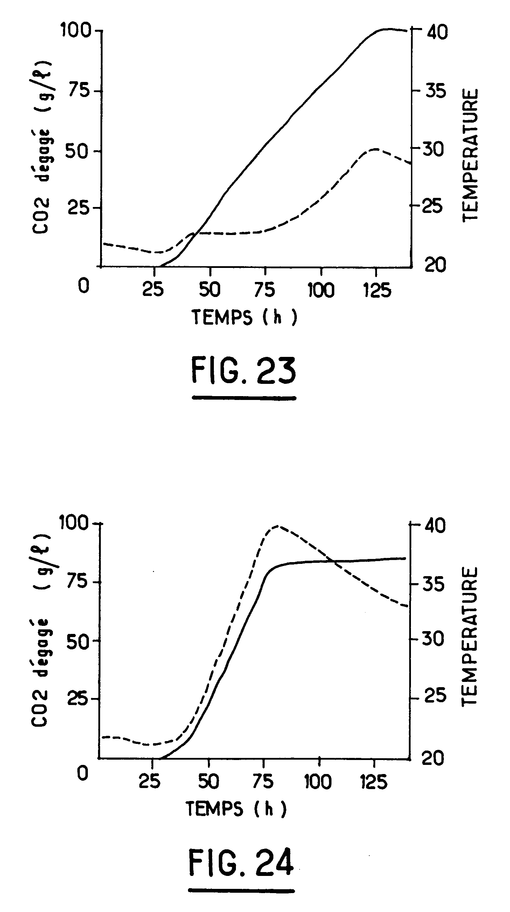

La figure 24 montre la faisabilité du calcul de masse volumique à partir de deux mesures de pression faites entre haut et bas de la partie immergée de la cuve. Il résulte des expérimentations industrielles réalisées que le compteur volumétrique est un bon capteur de cinétique fermentaire à l'échelle industrielle sous réserve de résoudre les problèmes liés aux condensations internes de vapeurs. Mais l'on pourrait utiliser n'importe quel dispositif de mesure de débit approprié à l'usage. Par ailleurs les capteurs de pression constituent de bons instruments de caractérisation de l'état d'avancement de la fermentation, notamment ce qui concerne le calcul de perte de poids ou de masse volumique. Ainsi, les expérimentations industrielles, viennent corroborer les bons résultats obtenus au niveau de l'expérimentation préalable de laboratoire, en ce qui concerne la faisabilité du procédé de prévision et de contrôle des fermentations selon la présente invention.

Il est clair que l'invention n'est nullement limitée à la forme et au mode de réalisation décrit ci-dessus, mais qu'elle englobe toutes les modifications et variantes, issues du même principe de base, à savoir l'évaluation des données initiales d'une fermentation, la réalisation des mesures des différents paramètres de la fermentation in situ, ainsi que le traitement des données ainsi mesurées pour l'expression des grandeurs physiques, les calculs cinétiques, la prévision des étapes finales et intermédiaires et la quantification de l'avancement de la fermentation.

Il est donc possible de prévoir dans une usine comportant un grand nombre de cuves de fermentation, l'instrumentation spécifique de chaque cuve et la mise en place d'un organe informatique centralisé assurant l'ensemble des traitements, calculs et prévisions, une telle solution, qui permet d'étendre progressivement le nombre de cuves prises en charge, a l'avantage de limiter au maximum les coûts d'investissement. Par ailleurs l'intérêt majeur du procédé est de permettre aux opérateurs d'accéder à tout instant à un maximum d'informations sur le déroulement de la fermentation alcoolique dans chaque cuve. Ces informations permettront la prise de certaines décisions relatives à la conduite du procédé. Les actions de réfrigération (en oenologie) ou de changement de la température de fermentation (en brasserie) sont les plus directement applicables.

Les applications du procédé concernent les techniques de fermentations alcooliques discontinues (dites batch) caractéristiques de la production des boissons fermentées, à savoir la bière, le vin, le cidre, ou les spiritueux et analogues, et dans certains cas du bio-éthanol. Sous réserves de quelques adaptations au niveau des logiciels et des modèles mathématiques et corrélations, ces applications peuvent s'étendre aux fermentations alcooliques réalisées en continu. Dans ce cas au lieu de prendre en compte le volume de $CO_2$ dégagé en fonction du temps, il faut partir des variations de volume du gaz carbonique dégagé par unité de temps.

Sur un plan plus général, il est possible de considérer que tous les procédés de fermentation anaérobie (ou micro-aérobie, c'est-à-dire supposant soit une saturation par l'air du milieu de culture initial, soit une micro-aération), générateurs de gaz, peuvent être contôles par un procédé mesurant en continu le volume de gaz produit. C'est le cas des procédés de production de méthane (le mélange $CO_2 + CH_4$ produit doit

alors être caractérisé, non pas par son débit total, mais par sa composition), et de production d'acétone-butanol (le mélange $H_2$ + $CO_2$ doit également être caractérisé par son débit et sa composition). C'est aussi le cas, sous certaines conditions biologiques (par exemple ajout d'urée dans le milieu de culture pour activer la production du $CO_2$) des procédés de fermentations lactiques: production de levains en cultures pures ou en cultures mixtes, utilisation de ces levains en fromagerie.

La description qui précède montre qu'on peut contrôler l'évolution de la fermentation par modification appropriée de la température à l'aide d'une loi de pilotage faisant intervenir la vitesse de dégradation des sucres.

Ainsi sur la figure 23, qui traite de l'évolution du $CO_2$ dégagé (continu) et de la température (pointillé) en fonction du temps, une consigne de vitesse maximale est fixée à $1g.l^{-1}.h^{-1}$. Une fois cette vitesse de consigne atteinte, la réfrigération maintient en fait constante, le $CO_2$ se dégageant linéairement en fonction du temps, soit en maintenant la température constante, soit en la laissant dériver vers le haut en fin de fermentation. Les courbes présentées sur la figure 24 sont des courbes témoins de la même fermentation sans aucun contrôle. On voit que la température s'élève de façon trop importante, ce qui se traduit par un arrêt de fermentation, le $CO_2$ final dégagé étant plus faible que sur la figure 23.

Les courbes de cette figure traduisent une première application concrète au pilotage des fermentations oenologiques par le contrôle de leur température. Grâce à l'acquisition en temps réel de la quantité de $CO_2$ dégagé, on peut calculer une vitesse instantanée de fermentation. La nouvelle loi de pilotage consiste à empêcher le dépassement d'une vitesse de consigne par l'envoi de frigories. Il s'agit là d'un nouveau moyen de contrôle des températures, non plus basé sur la fixation, a priori, d'un point de consigne de ce paramètre, mais adapté aux caractéristiques mesurées en temps réel de chaque fermentation. Ce système permet une économie de frigories, une amélioration de la productivité des fermenteurs ou bio-réacteurs, ainsi qu'une augmentation de la faisabilité de la réfrigération des moûts en fermentation. Dans l'exemple d'application de la nouvelle loi de pilotage présentée aux figures 23 et 24, on utilise, 15 comme spécifié ci-dessus, une consigne de vitesse maximale de $1.gl^{-1}.h^{-1}$ de $CO_2$ dégagé, mais il est bien évident que ces consignes de vitesse maximale doivent être adaptées à chaque grand type de situation oenologique.

Quand aux corrélations établies entre mesures physiques et teneurs en éléments chimiques,leur exploitation intéresse un certain nombre de domaines.

L'exploitation des corrélations entre paramètres permet de déterminer, en ligne, l'état d'avancement des fermentations alcooliques,par détermination de la teneur en sucres et/ou éthanol à un instant donné, et en la (les) comparant à la (aux) valeurs initiales.

Un autre domaine d'exploitation de ces corrélations est l'établissement de cinétiques, telles que celles de production de gaz carbonique, dégradation des sucres, formation d'éthanol, et autres. Ces déterminations de cinétiques sont directement utilisées pour une fonction d'alarme, de diagnostic, et de contrôle.

Un autre domaine d'exploitation de ces corrélations est le pilotage des fermentations alcooliques à l'aide d'algorithmes spécifiques reposant sur la détermination en ligne des teneurs en éthanol produit et en sucres résiduels, et des cinétiques associées.

TABLEAU 1

Ecude de l'homogénéicé d'une fermentacion

(Fermentacion en cave)

2700 litres - Domaine du Chapitre 1985

| Numéro | | 1 | 2 | 3 | 4 | 5 | 6 | 7 | 8 | 9 | 10 | 11 |
|---|---|---|---|---|---|---|---|---|---|---|---|---|
| Heures | | 0 | 4 | 8 | 12 | 20 | 24 | 28 | 32 | 36 | 44 | 48 |
| Biomasse totale | | $0,248 \cdot 10^8$ | $0,454 \cdot 10^8$ | $1,363 \cdot 10^8$ | $2,344 \cdot 10^8$ | $3,088 \cdot 10^8$ | $3,125 \cdot 10^8$ | $2,675 \cdot 10^8$ | $3,163 \cdot 10^8$ | $2,298 \cdot 10^8$ | $2,338 \cdot 10^8$ | //// |
| Alcool g/l | A | 0 | 0 | 7,1 | 12,2 | 28,5 | 34,4 | 41,1 | 40,3 | 51,0 | 62,4 | 68,3 |
| | B | 0 | 0 | 5,5 | 11,1 | 25,3 | (23,3) | 34,8 | 45,4 | 54,9 | 62,0 | 70,7 |
| | C | 0 | 0 | 4,7 | 11,1 | 18,8 | 32,3 | 35,6 | 41,1 | 53,3 | 54,0 | 68,3 |
| moyenne | | 0 | 0 | 5,8 | 11,5 | (23,5) | 33,6 | (37,2) | 42,3 | 53,1 | 62,8 | 69,1 |
| Glycé-roi g/l | A | 1,5 | 1,6 | 1,5 | 1,9 | 4,4 | 5,1 | 5,9 | 4,3 | 5,6 | 5,9 | 6,1 |
| | B | 1,3 | 1,4 | 1,9 | 2,1 | 4,1 | (3,2) | 4,0 | 5,0 | 5,9 | 6,0 | 6,5 |
| | C | 1,1 | 1,4 | 1,4 | 2,2 | 2,7 | 4,6 | 4,6 | 4,5 | 6,0 | 5,9 | 6,1 |
| moyenne | | 1,3 | 1,47 | 1,60 | 2,07 | (3,7) | 4,85 | (4,8) | 4,60 | 5,83 | 5,93 | 6,23 |
| Sucre g/l | A | 185,0 | 189,0 | 172,0 | 162,0 | 125,0 | 113,0 | 100,0 | 74,7 | 69,4 | 46,5 | 39,0 |
| Indice de réfraction corrigé à 20°C (%) | A | 1 3641 | 1 3643 | 1 3628 | 1 3594 | 1 3573 | 1 3557 | 1 3538 | 1 3518 | 1 3480 | 1 3461 | 1 3458 |
| | B | 1 3642 | 1 3637 | 1 3628 | //// | 1 3576 | 1 3569 | 1 3537 | 1 3514 | 1 3484 | //// | 1 3462 |
| | C | 1 3642 | 1 3635 | 1 3630 | 1 3597 | 1 3556 | 1 3568 | 1 3542 | 1 3520 | 1 3486 | //// | 1 3462 |
| | D | 1 3643 | 1 3635 | 1 3632 | 1 3605 | 1 3564 | 1 3568 | 1 3542 | 1 3515 | 1 3490 | 1 3462 | 1 3458 |
| | E | 1 3643 | 1 3638 | 1 3632 | 1 3595 | 1 3554 | 1 3558 | 1 3540 | 1 3522 | 1 3488 | 1 3464 | 1 3468 |
| moyenne | | 1 3642 | 1 3636 | 1 3630 | 1 3598 | 1 3565 | 1 3564 | 1 3540 | 1 3518 | 1 3486 | 1 3462 | 1 3461 |
| écart-type σ | | $1,32 \cdot 10^{-4}$ | $1,87 \cdot 10^{-4}$ | $2,00 \cdot 10^{-4}$ | $5,00 \cdot 10^{-4}$ | $9,85 \cdot 10^{-4}$ | $5,96 \cdot 10^{-4}$ | $2,29 \cdot 10^{-4}$ | $3,16 \cdot 10^{-4}$ | $3,91 \cdot 10^{-4}$ | $2,24 \cdot 10^{-4}$ | $5,12 \cdot 10^{-4}$ |

(alcool): moyenne mise entre parenthèses lorsque σ est supérieur à 3
(glycérol): moyenne mise entre parenthèses lorsque σ est supérieur à 0,5
les valeurs d'alcool ou de glycérol A, B ou C mise entre parenthèses ne sont pas
comptées dans la valeur moyenne d'alcool ou de glycérol
(%) : la correction est faite en utilisant les tables de correction par la température
appliquées à un moût non fermenté

TABLEAU 2

Etude de l'homogénéité d'une fermentation

(fermentation en cave ; 45 hl ; vendange rouge chauffée)

| TEMPS DE PRELEVEMENT ET DE MESURE | | BAS DE CUVE | MILIEU DE CUVE | HAUT DE CUVE |
|---|---|---|---|---|
| 12/09/86 à 23 h 00 | S | 180,3 | 180,3 | 180,3 |
| | $\frac{dS}{dt}$ | - | - | - |
| | A | 0,10 | 0,10 | 0,10 |
| | G | 0,14 | 0,07 | 0,07 |
| | d | 1084,0 | 1079,4 | 1079,4 |
| | θ | 21,9 | 22,1 | 22,3 |
| | n | | | |
| | θ | | | |
| 13/09/86 à 09 h 00 | S | 174,2 | 180,3 | 178,9 |
| | $\frac{dS}{dt}$ | 0,6 | 0 | 0,1 |
| | A | 0,35 | 0,27 | 0,29 |
| | G | 0,92 | 0,42 | 0,46 |
| | d | 1076,8 | 1077,6 | 1077,6 |
| | θ | 22,3 | 22,1 | 22,15 |
| | n | 1360,8 | 1361,2 | 1361,6 |
| | θ | 20 | 20 | 20 |
| 13/09/86 à 23 h 00 | S | 149 | 149 | 147,8 |
| | $\frac{dS}{dt}$ | 1,8 | 2,23 | 2,22 |
| | A | 1,58 | 1,68 | 1,90 |
| | G | 2,41 | 2,29 | 2,37 |
| | d | 1065,0 | 1066,2 | 1065,7 |
| | θ | 22,7 | 23 | 22,85 |
| | n | 1362,7 | 1354,8 | 1355,7 |
| | θ | 20,5 | 22 | 20,5 |

S = teneur en sucres (g/l)

$\frac{dS}{dt}$ = vitesse de dégradation des sucres (g/l.h)

A = teneur en éthanol(% V)
G = teneur en glycérol (g/l)
d = densité
n = indice de réfraction
θ = température associée à la mesure physique

| TEMPS DE PRELEVEMENT ET DE MESURE | | BAS DE CUVE | MILIEU DE CUVE | HAUT DE CUVE |
|---|---|---|---|---|
| 14/09/86 à 09 h 00 | S | 112 | 113,3 | 112,8 |
| | $\frac{dS}{dt}$ | 3,7 | 3,6 | 3,5 |
| | A | 3,64 | 3,62 | 3,43 |
| | G | 3,71 | 3,61 | 3,69 |
| | d | 1051,1 | 1053,8 | 1051,0 |
| | θ | 22,3 | 22,75 | 22,3 |
| | n | 1353,6 | 1352,6 | 1358,7 |
| | θ | 21 | 21,5 | 21 |
| 13/09/86 à 23 h 00 | S | 79,3 | 80,8 | 80,2 |
| | $\frac{dS}{dt}$ | 2,3 | 2,3 | 2,3 |
| | A | 5,40 | 5,20 | 5,10 |
| | G | 4,63 | 4,58 | 4,74 |
| | d | 1036,3 | 1037,4 | 1037,5 |
| | θ | 21,3 | 22,0 | 21,75 |
| | n | 1348,1 | 1355,2 | 1349,0 |
| | θ | 20 | 20,5 | 20 |
| 15/09/86 à 09 h 00 | S | 57,9 | 60,0 | 58,9 |
| | $\frac{dS}{dt}$ | 2,2 | 2,1 | 2,1 |
| | A | 6,00 | 6,10 | 6,78 |
| | G | 5,37 | 5,16 | 5,16 |
| | d | – | 1027,2 | 1027,3 |
| | θ | – | 22,3 | 22,35 |
| | n | 1344,9 | 1349,6 | 1348,0 |
| | θ | 20 | 20,5 | 20 |
| 15/09/86 à 23 h 00 | S | 35,2 | 37,1 | 36,0 |
| | $\frac{dS}{dt}$ | 1,6 | 1,6 | 1,6 |
| | A | 8,09 | 8,10 | 8,20 |
| | G | 5,80 | 5,76 | 5,75 |
| | d | 1019,3 | 1017,3 | 1016,8 |
| | θ | 22,4 | 22,3 | 22,1 |
| | n | 1346,7 | 1347,0 | 1346,9 |
| | θ | 20 | 22,5 | 20 |

S = teneur en sucres (g/l)

$\frac{dS}{dt}$ = vitesse de dégradation des sucres (g/l.h)

A = teneur en éthanol (% V)

G = teneur en glycérol(g/l)

d = densité

n = indice de réfraction

θ = température associée à la mesure physique

| TEMPS DE PRELEVEMENT ET DE MESURE | | BAS DE CUVE | MILIEU DE CUVE | HAUT DE CUVE |
|---|---|---|---|---|
| 16/09/86 à 09 h 00 | S | 20 | 21,6 | 20 |
| | $\frac{dS}{dt}$ | 1,5 | 1,6 | 1,6 |
| | A | 8,92 | 8,99 | 8,73 |
| | G | 6,13 | 6,02 | 6,13 |
| | d | 1013,2 | 1010,5 | 1010,0 |
| | θ | 23,2 | 23,4 | 23,4 |
| | n | 1345,9 | 1347,1 | 1338,1 |
| | θ | 20 | 20,5 | 20 |
| 16/09/86 à 23 h 00 | S | 2,3 | 2,8 | 2,6 |
| | $\frac{dS}{dt}$ | 1,3 | 1,3 | 1,2 |
| | A | 9,81 | 9,85 | 9,86 |
| | G | 6,45 | 6,47 | 6,43 |
| | d | 1002,3 | 1001,3 | 1000,7 |
| | θ | 24,7 | 25,3 | 25,0 |
| | n | 1338,9 | 1348,5 | 1340,8 |
| | θ | 20 | 20 | 20 |
| 17/09/86 à 09 h 00 | S | 1,0 | 1,0 | 1,0 |
| | $\frac{dS}{dt}$ | 0,1 | 0,2 | 0,2 |
| | A | 9,83 | 9,82 | 9,57 |
| | G | 6,45 | 6,45 | 6,45 |
| | d | 1000,4 | 999,4 | 999,7 |
| | θ | 24,5 | 24,7 | 24,9 |
| | n | 1341,0 | 1342,6 | 1338,9 |
| | θ | 20 | 20 | 20 |
| 17/09/86 à 23 h 00 | S | 0,95 | 0,95 | 0,95 |
| | $\frac{dS}{dt}$ | 0 | 0 | 0 |
| | A | 9,88 | 9,77 | 10,17 |
| | G | 6,50 | 6,43 | 6,56 |
| | d | 999 | 999,6 | 999,5 |
| | θ | 24,2 | 24,5 | 24,3 |
| | n | 1342,4 | 1341,8 | 1341,8 |
| | θ | 20,5 | 21,5 | 20,5 |
| 18/09/86 à 09 h 00 | S | 0,95 | 1,0 | 1,0 |
| | $\frac{dS}{dt}$ | 0 | 0 | 0 |
| | A | 9,92 | 9,97 | 10,00 |
| | G | 6,76 | 6,22 | 6,38 |
| | d | 999,0 | 1000,2 | 999,0 |
| | θ | 23,1 | 23,6 | 23,6 |
| | n | 1340,5 | 1339,9 | 1341,3 |
| | θ | 20 | 20 | 20 |

S = teneur en sucres (g/l)

$\frac{dS}{dt}$ = vitesse de dégradation des sucres (g/l.h)

A = teneur en éthanol (% V)

G = teneur en glycérol (g/l)

d = densité

n = indice de réfraction

θ = température associée à la mesure physique

## Revendications

1. Procédé de prévision et de contrôle in situ des fermentations alcooliques, caractérisé en ce que l'on mesure à intervalles de temps donnés au moins un paramètre choisi parmi la densité du milieu de fermentation, l'indice de réfraction dudit milieu, le volume de gaz carbonique produit par ledit milieu et

13

la perte de poids du milieu de fermentation, en ce que l'on détermine, à partir de ces paramètres, les concentrations en éthanol et en sucres résiduels, concentrations à partir desquelles on peut déterminer l'avance et l'évolution de la fermentation alcoolique, diagnostiquer les évolutions incorrectes (arrêts ou accidents de fermentation) et contrôler cette dernière de façon à corriger toute évolution incorrecte du déroulement de ladite fermentation.

2. Procédé selon la revendication 1, caractérisé en ce que l'on détermine la cinétique, instantanée ou globale, de la fermentation, par exemple la vitesse de consommation des sucres, la vitesse de production d'éthanol et la vitesse de production de $CO_2$.

3. Procédé selon la revendication 2, caractérisé en ce que la détermination de la cinétique instantanée ou globale de la fermentation est mise à profit pour contrôler automatiquement l'évolution de la fermentation, en particulier en provoquant des modifications appropriées de la température.

## Claims

1. Process for the on-line preview and control of alcoholic fermentations, characterized in that a measurement is taken, at given intervals, of at least one parameter chosen from among the following: the specific gravity of the fermentation medium, the refractory index of the said medium, the volume of carbon dioxide produced by the said medium and the weight loss of the fermentation medium, and in that a determination is made from these parameters of the concentrations in ethanol and residual sugars, concentrations from which the progression and evolution of alcoholic fermentation can be determined, incorrect evolutions diagnosed (interruptions of or problems with fermentation) and the latter controlled so as to correct any unsatisfactory developments in the said fermentation.

2. Process according to claim 1, characterized in that a determination is made of the instantaneous or global kinetics of the fermentation, for instance the rate of consumption of sugars, the rate of ethanol production and the rate of $CO_2$ production.

3. Process according to claim 2, characterized in that determination of the instantaneous or global kinetics of the fermentation is exploited to automatically control the evolution of the fermentation, particularly by causing appropriate modifications of the temperature.

## Patentansprüche

1. Verfahren zur *in situ* Vorausberechnung und Kontrolle alkoholischer Gärungen, dadurch gekennzeichnet, daß in gegebenen Zeitintervallen wenigstens ein Parameter gemessen wird, ausgewählt aus der Dichte des Gärungsmediums, des Brechungsindex dieses Mediums, des durch das Medium erzeugten Volumens an Kohlensäuregas und des Gewichtsverlusts des Gärungsmediums, indem ausgehend von diesen Parametern die Konzentrationen an Ethanol und verbliebenen Zuckern bestimmt werden, Konzentrationen, von denen ausgehend sich Fortschreiten und Entwicklung der alkoholischen Gärung bestimmen lassen, fehlerhafte Entwicklungen (Stillstand oder Komplikationen bei der Gärung) feststellen lassen und letztere sich in einer Weise kontrollieren lassen, daß alle fehlerhaften Entwicklungen im Ablauf der Gärung zu korrigieren sind.

2. Verfahren nach Anspruch 1, dadurch gekennzeichnet, daß die augenblickliche oder gesamte Kinetik der Gärung bestimmt wird, beispielsweise die Geschwindigkeit des Verbrauchs der Zucker, die Geschwindigkeit der Ethanol-Produktion und die Geschwindigkeit der $CO_2$-Produktion.

3. Verfahren nach Anspruch 2, dadurch gekennzeichnet, daß die Bestimmung der augenblicklichen oder gesamten Kinetik der Gärung genutzt wird für die automatische Kontrolle der Gärungsentwicklung, insbesondere durch Hervorrufen geeigneter Temperaturänderungen.

14

FIG. 1

FIG. 2

FIG. 3

FIG. 4

FIG. 5

FIG. 6

EP 0 271 380 B1

FIG. 7

FIG. 8

FIG.9

FIG.10

23

FIG. 12

FIG. 11

11_12_13 : CAPTEUR DE PRESSION

14 : CAPTEUR DE TEMPERATURE

A_B_C_D_E : POINTS DE PRLEVEMENT ET D'ENREGISTREMENT
DE LA TEMPERATURE

15 : COMPTEUR A GAZ

## FIG.13

FIG. 14 A

FIG. 14 B

EP 0 271 380 B1

[ALCOOL] calculée ( % V )

□ : TENEUR EN ALCOOL CALCULEE
A PARTIR DE LA MESURE
D'INDICE DE REFRACTION.

○ : TENEUR EN ALCOOL CALCULEE
A PARTIR DE LA MESURE DE
DENSITE.

[ALCOOL] analyse ( % V )

FIG. 15

[ALCOOL] calculée ( % V )

□ : DITO FIG. 15
○ : DITO FIG. 15

FIG. 16

[ALCOOL] analyse ( % V )

27

CONCENTRATIONS (g/ℓ)

CONCENTRATIONS
CALCULEES.

+ + + CONCENTRATIONS
ANALYSEES.

TEMPS (h)

FIG. 18

[ALCOOL] calculée (%V)

□ : DITO FIG. 15
○ : DITO FIG. 15

[ALCOOL] analyse (%V)

FIG. 17

28

FIG. 19

FIG. 20

FIG. 22

FIG. 21

FIG. 23

FIG. 24